# EUROPEAN PATENT APPLICATION

(11) **EP 1 810 962 A1**
(43) Date of publication of application: **25.07.2007**
(21) Application number: 07000720.8
(22) Date of filing: 15.01.2007
(51) Int. Cl.: C07C 213/10, C07C 215/54

(54) **A process for the purification of tolterodine**

(30) Priority: 24.01.2006 IT MI20060110
(71) Applicant: Dipharma Francis S.r.l., 20021 Baranzate di Bollate MI (IT)
(72) Inventor: Allegrini, Pietro, 20097 San Donato Milanese Milano (IT); Mantegazza, Simone, 20144 Milano (IT); Razzetti, Gabriele, 20099 Sesto San Giovanni Milano (IT)
(74) Representative: Minoja, Fabrizio

(57) **Abstract**

Process for the purification of tolterodine comprising reacting racemic tolterodine with sulfuric acid to obtain an addition salt and racemic tolterodine acid sulfate in the crystalline form.

## Description

### SUMMARY

Tolterodine crystalline forms and the use thereof in a process for its purification.

### FIELD OF THE INVENTION

The present invention relates to a process for the purification of racemic tolterodine, and a novel crystalline form of salts thereof.

### TECHNOLOGICAL BACKGROUND

Tolterodine, namely N,N-diisopropyl-3-(2-hydroxy-5-methylphenyl)-3-phenylpropanamine, having formula, 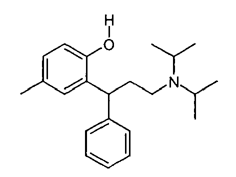 is marketed in the form of S(+) tolterodine tartrate as a medicament for the treatment of urinary incontinence. Its preparation is disclosed, for example, in EP 325571 and it involves formation of impurities difficult to remove. There is therefore the need for an improved process for the purification of Tolterodine, which is easy to carry out and makes use of inexpensive reagents.

### SUMMARY OF THE INVENTION

It has now been found a process for the purification of tolterodine, which comprises the preparation of an addition salt of racemic tolterodine with sulfuric acid and its recovery in the crystalline form. The novel process affords S(+) tolterodine tartrate in a high purity, suitable to fulfil regulatory requirements.

### BRIEF DISCLOSURE OF THE FIGURE

The novel crystalline forms were characterized with the known XRPD technique (X-Ray Powder Diffraction). X-Ray Diffraction Spectra (XRPD) were recorded with a θ/θ automatic diffractometer (Ital-Structures), under the following operative conditions: CuKα radiation (λ= 1.5418 A), scanning with angular interval 2-40°, with angular step of 0.03° for 1 sec.

Figure 1. XRPD Spectrum of racemic tolterodine hydrogen sulphate salt.

Figure 2. XRPD Spectrum of racemic tolterodine free base.

According to present invention, when a particles sample is stated to have mean diameter, referred to as D[4,3], higher than X µm, this means that the mean volume of the particles constituting the sample is higher than the volume of a spherical particle with diameter X.

According to the present invention, the term "particle" means a single entity, both as a single and geminate crystal.

Particles size, namely mean diameter "D[4,3]" values, were determined with the known laser light scattering technique using a Malvern Mastersizer MS 1 instrumentation under the following operative conditions:
- 300RF mm lens with of 2.4 mm laser beam length;
- sample of 500 mg dispersed in 10 ml of hexane (reagent ACS) with 1% SPAN 85^{®}, without presonication, and 2500 rpm stirring rate.

### DETAILED DISCLOSURE OF THE INVENTION

An object of the present invention is a process for the purification of racemic tolterodine to obtain a product having a purity degree equal to or higher than 99%, which process comprises:
a) reacting racemic tolterodine with sulfuric acid to obtain an addition salt;
b) recovering the addition salt; and
c) contacting said salt with a basic agent and recovering racemic tolterodine base.

The reaction of racemic tolterodine with sulfuric acid to form the addition salt, i.e. racemic tolterodine acid sulfate, can be carried out by contacting crude racemic tolterodine with a sulfuric acid aqueous solution, typically having a concentration lower than 50%, preferably approx. of 5 - 25%, more preferably approx. 10 - 20%. Alternatively, tolterodine acid sulfate can be obtained first preparing a dispersion of crude racemic tolterodine in concentrated sulfuric acid and then diluting with water.

The crude racemic tolterodine starting product can be for example racemic tolterodine from an industrial batch.

Typically, the concentration of crude racemic tolterodine in the reaction mixture approx. ranges from 5 to 15%, and the molar ratio of sulfuric acid to tolterodine base approx. ranges from 1:1 to 10:1, preferably approx. from 1:1 to 1.5:1.

The reaction temperature usually ranges from room temperature to 60°C.

The resulting tolterodine acid sulfate is recovered, washed and dried. The separation from the reaction mixture can be carried out, for example, by cooling or dilution, followed by recovery according to a known technique, such as filtration or centrifugation. If desired, the resulting crude can be washed with water.

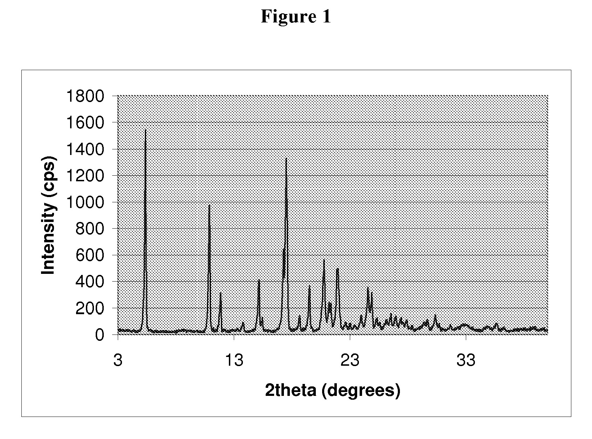

The resulting addition salt of racemic tolterodine with sulfuric acid is in a crystalline form having an XRPD spectrum substantially as reported in figure 1, wherein the most intense diffraction peaks fall at about 5.3; 10.8; 11.8; 15.1; 17.5; 19.5; 20.7; 21.1; 21.9 and 24.5° in 2θ. This is a novel compound and is object of the present invention.

The addition salt of racemic tolterodine with sulfuric acid obtained according to the present process has a purity degree typically equal to or higher than 99%, preferably equal to or higher than 99.5%. The resulting product has therefore the purity requirements necessary to fulfil the regulatory requirements, and can be transformed into the active ingredient without further purification steps, which is an improvement over the known processes.

The resulting addition salt is contacted with a basic agent, which can be an organic or inorganic compound, in water, to obtain racemic tolterodine free base.

Preferred examples of inorganic basic agents are ammonia, either gas or in aqueous solution, and caustic sodium hydroxide, either solid or dissolved in water, in particular caustic sodium hydroxide, preferably in an about 50% aqueous solution.

Preferred examples of organic basic agents are primary, secondary and tertiary amines, as monomethylamine, triethylamine, dicyclohexylamine, in particular monomethylamine, preferably in aqueous solution.

The resulting racemic tolterodine free base, which can be recovered by extraction in an water-immiscibile organic solvent, preferably toluene, has a purity degree equal to or higher than 99%, in particular equal to or higher than 99.5%.

The invention also provides a process for the preparation of S(+) tolterodine tartrate, having a purity equal to or higher than 99.5%, typically equal to or higher than 99.9%, and with a potentiometric assay ranging from 99 to 101 %, which process comprises reacting racemic tolterodine free base, as obtainable according to present invention, with L(+) tartaric acid. The reaction can be carried out for example in ethanol according to EP 325 571.

According to the invention, (S)- tolterodine tartrate is obtained as particles typically having a diameter mean D[4,3] ranging from 20 to 200 µm, preferably ranging from 50 and 150 µm. Such particle size allows the easy recovery of the product when, at the end of its preparation, is subjected to filtration or centrifugation, thereby remarkably reducing the time necessary for this operation and for the subsequent drying.

If desired, at the end of the process described above, particles can be subjected to conventional fine-grinding or micronisation, to obtain particles having lower mean diameter D[4,3], typically equal to or lower than 5 µm, preferably lower than or equal to 2 µm.

If desired, tolterodine free base as obtained above can be crystallized from n-hexane to obtain racemic tolterodine free base in the crystalline form, having an XRPD spectrum substantially as reported in Figure 1, wherein the most intense diffraction peaks fall at about 9.0; 10.0; 12.4; 16.8; 17.6; 18.5; 20.1; 22.9; 24.2 and 25.8° in 2θ. This is a novel compound and is a further object of the present invention.

The following examples illustrate the invention.

### EXAMPLE 1. Preparation of racemic tolterodine hydrogen sulfate

In a 250 ml 4-necked round-bottom flask, equipped with mechanical stirrer, reflux condenser and thermometer, crude racemic tolterodine (50.0 g, 0.154 mol) and 10% sulfuric acid (150 g, 0.154 mol) are dropped. The dispersion is vigorously stirred and cooled to 10-15°C. The suspended solid is filtered and washed with water cooled to 10-15°C (50 ml) three times, then the solid is dried under vacuum in a static dryer at 60°C for 18 h. 62.5 g of product are obtained; 96% yield. The crystalline solid has an XRPD spectrum substantially as reported in Figure 1, wherein the most intense diffraction peaks fall at about 5.3; 10.8; 11.8; 15.1; 17.5; 19.5; 20.7; 21.1; 21.9 and 24.5° in 2θ.

### EXAMPLE 2. Preparation of tolterodine tartrate

Tolterodine sulfate salt (100 g, 0.236 mol) is suspended in water (250 ml) and toluene (250 ml) in a 3 1 4-necked round-bottom flask, equipped with mechanical stirrer, reflux condenser and thermometer. The vigorously stirred suspension is added with a 41 % monomethylamine solution (26.8 g, 0.354 mol), and phases are separated after complete dissolution of the salt. The organic phase is concentrated to small volume under reduced pressure and taken up in 95% ethanol (1000 ml). The resulting racemic tolterodine base has a purity higher than 99.5% by HPLC analysis. A solution of L(+)-tartaric acid is added to precipitate the salt. The mixture is heated to complete dissolution of the salt (about 80°C) and subsequently seeded with some crystals of the product and slowly cooled. (+)-Tolterodine tartrate precipitates and is recovered by filtration, then dried under vacuum in oven at 60°C, to afford 50 g of (+)-tolterodine tartrate with a purity higher than 99.5% (45% yield, on racemic tolterodine sulphate salt). The product is in form of particles having a mean diameter D[4,3] approx. ranging from 50 to 150 µm.

### EXAMPLE 3. Crystalline racemic tolterodine free base

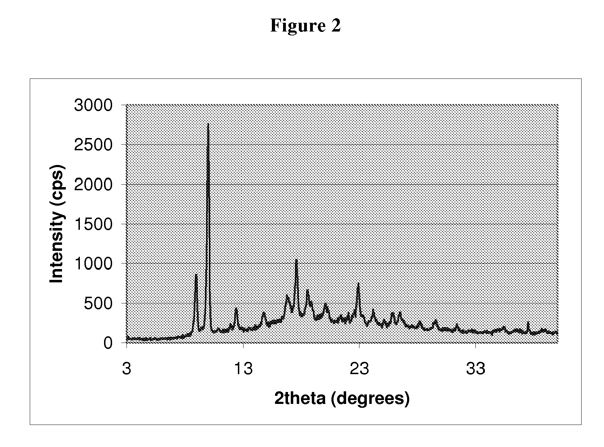

Racemic tolterodine hydrogen sulfate, obtained in Example 1, is dissolved in water, treated with a 50% caustic sodium hydroxide aqueous solution and extracted with n-hexane. The organic phase is separated, dried and concentrated to a volume of approx. 150 ml. The solution is cooled to a temperature f about 0 - 5°C and kept at this temperature under stirring for approx. 3 hours. The precipitated solid is filtered and dried at about 20 -35°C under vacuum, to afford crystalline racemic tolterodine free base, having an XRPD spectrum substantially as reported in Figure 2, wherein the most intense diffraction peaks fall at about 9.0; 10.0; 12.4; 16.8; 17.6; 18.5; 20.1; 22.9; 24.2 and 25.8° in 2θ.

## Claims

1. A process for the purification of racemic tolterodine to obtain a product having a purity degree equal to or higher than 99%, which process comprises:
a) reacting racemic tolterodine with sulfuric acid to obtain an addition salt;
b) recovering the addition salt; and
c) contacting said salt with a basic agent and recovering racemic tolterodine base.

2. The process as claimed in claim 1, wherein racemic tolterodine is reacted with sulfuric acid by contacting a crude containing racemic tolterodine with a sulfuric acid aqueous solution.

3. The process as claimed in claim 2, wherein the concentration of the acid is lower than 50%.

4. The process as claimed in claim 1, wherein tolterodine acid sulfate is obtained by dispersing crude racemic tolterodine in concentrated sulfuric acid and subsequently diluting it with water.

5. The process as claimed in any one of the above claims, wherein the concentration of crude racemic tolterodine in the reaction mixture approx. ranges from 5 to 15%.

6. The process as claimed in any one of the above claims, wherein the molar ratio of sulfuric acid to tolterodine base approx. ranges from 1:1 to 10:1.

7. The process as claimed in claim 1, wherein the basic agent is gaseous or aqueous ammonia, caustic sodium hydroxide solid or dissolved in water, a primary, secondary or tertiary amine.

8. The process as claimed in claim 1, wherein the basic agent is an approx. 50% caustic sodium hydroxide aqueous solution, an ammonia aqueous solution or a monomethylamine aqueous solution.

9. The process as claimed in claim 1, wherein tolterodine free base is recovered by extraction in a water-immiscibile organic solvent.

10. The process as claimed in claim 9, wherein the solvent is toluene.

11. The process as claimed in any one of the above claims, further comprising the reaction with L(+)-tartaric acid to obtain S(+)-tolterodine tartrate, having a purity equal to or higher than 99.5% and a potentiometric assay ranging from 99 to 101%.

12. Racemic tolterodine acid sulfate in the crystalline form.

13. Tolterodine acid sulfate, as claimed in claim 12, having an XRPD spectrum wherein the most intense diffraction peaks fall at about 5.3; 10.8; 11.8; 15.1; 17.5; 19.5; 20.7; 21.1; 21.9 and 24.5° in 2θ. +2

14. Tolterodine acid sulfate, as claimed in claim 13, having an XRPD spectrum substantially as illustrated in Figure 1.
